# FASCICULE DE BREVET EUROPEEN

(11) **EP 1 493 378 B1**
(45) Date de publication et mention de la délivrance du brevet: **10.09.2008**
(21) Numéro de dépôt: 04291477.0
(22) Date de dépôt: 11.06.2004
(51) Int. Cl.: G11B 20/24, A61B 1/05, H04N 7/18, A61B 1/04, G02B 23/00

(54) **Vidéoendoscope avec processeur vidéo multistandard déporté pour réduction de bruit électronique.**
Videoendoskope mit abgelegenem multistandard Videoprozessor um Elektronisches Rauschen zu reduzieren.
Video-endoscope with remote multistandard videoprocesseur to reduce electronic noise.

(30) Priorité: 01.07.2003 FR 0307950
(43) Date de publication de la demande: 05.01.2005
(73) Titulaire: Tokendo, 13600 La Ciotat (FR)
(72) Inventeur: Rovegno, Jean, 13600 La Ciotat (FR)
(74) Mandataire: Bentz, Jean-Paul

(56) Documents cités:
- WO-A-93/25137
- FR-A- 2 785 132
- US-A- 5 335 662
- US-A- 5 614 943
- US-A- 5 702 345
- US-A1- 2002 171 733
- US-A1- 2003 018 237
- US-B1- 6 432 047

## Description

La présente invention concerne un processeur vidéo pour sonde vidéoendoscopique ou pour caméra vidéo connectable à un endoscope.

Elle s'applique notamment, mais non exclusivement à l'endoscopie à vocation médicale, ainsi qu'à l'endoscopie à vocation industrielle.

On désigne par les termes endoscope ou fibroscope une sonde souple ou rigide, destinée à être introduite dans une cavité obscure et permettant à son utilisateur d'observer à travers un oculaire l'image d'une cible située dans la cavité. A cet effet, une telle sonde intègre un dispositif d'illumination de la cible et un dispositif optique fournissant à l'utilisateur une image de la cible. Le dispositif optique comprend un objectif distal, un dispositif de transport d'images soit de nature rigide constitué d'une série de lentilles, soit de nature souple constitué d'un faisceau de fibres optiques ordonnées, et un oculaire proximal dans lequel l'utilisateur peut observer l'image de la cible. Le dispositif d'illumination comprend généralement un faisceau de fibres d'éclairage dont l'extrémité distale, convenablement orientée à proximité de l'objectif distal, illumine la cible quand son extrémité proximale est connectée à un générateur de lumière.

On désigne par le terme vidéoendoscope une sonde souple ou rigide permettant à son utilisateur d'observer sur un écran vidéo l'image d'une cible située dans une cavité obscure. A cet effet, un vidéoendoscope comprend un dispositif d'illumination de la cible identique à celui d'un endoscope ou d'un fibroscope et un dispositif optoélectronique fournissant à l'utilisateur une image vidéo de la cible. Un vidéoendoscope peut soit résulter de la connexion de l'oculaire d'un endoscope ou d'un fibroscope sur l'objectif d'une caméra d'endoscopie, soit présenter une architecture spécifique caractérisant une sonde vidéoendoscopique et comprenant :
- un embout distal logeant un dispositif optoélectronique comprenant notamment un capteur CCD présentant une surface photosensible sur laquelle l'objectif auquel il est associé forme une image,
- un tube d'inspection, le plus souvent de nature souple, dont l'extrémité distale est solidaire de l'embout distal,
- une poignée de commande solidaire de l'extrémité proximale du tube d'inspection,
- un tube ombilical souple de raccordement dont l'extrémité distale est solidaire de la poignée de commande et dont l'extrémité proximale est destinée à être raccordée à un coffret externe intégrant notamment un générateur de lumière et une source d'alimentation électrique,
- un faisceau de fibres d'éclairage logé dans le tube ombilical, dans la poignée de commande, puis dans le tube d'inspection et dont l'extrémité distale, logée dans l'embout distal, illumine la cible lorsque son extrémité proximale est connectée à un générateur de lumière,
- un processeur vidéo transformant en un signal vidéo utile le signal électrique délivré par le capteur CCD distal auquel il est relié par un câble électrique multiconducteurs, et dont la synchronisation est réglée en fonction de la longueur dudit câble et de la structure du microcircuit d'interface du capteur CCD,
- un panneau de commande permettant notamment de paramétrer le fonctionnement du processeur vidéo en fonction de la température de couleur de l'éclairement de la cible par l'extrémité distale du faisceau de fibres d'éclairage de la sonde vidéoendoscopique, et
- un moniteur vidéo relié au processeur vidéo et implanté, de façon préférentielle à proximité immédiate de la poignée de commande.

Comme les fibroscopes, les sondes vidéoendoscopiques souples peuvent comprendre en outre un béquillage distal articulé permettant de modifier l'orientation de l'embout distal de la sonde, la poignée de commande intégrant alors des moyens mécaniques ou électromécaniques permettant à l'utilisateur d'actionner le béquillage distal.

Par ailleurs, une caméra d'endoscope comporte les éléments suivants :
- une tête de caméra logeant un dispositif optoélectronique comprenant notamment un capteur CCD présentant une surface photosensible sur laquelle est formée l'image délivrée par l'objectif à mise au point réglable auquel il est associé, ainsi qu'un dispositif de verrouillage permettant de fixer sur l'objectif la bonnette d'un endoscope ou d'un fibroscope,
- un tube ombilical souple de raccordement dont l'extrémité distale est solidaire de la tête de caméra et dont l'extrémité proximale est destinée à être raccordée à un coffret externe dans lequel est généralement logé le processeur vidéo,
- un processeur vidéo transformant en un signal vidéo utile le signal électrique délivré par le capteur CCD de la tête de caméra à laquelle il est relié par un câble électrique multiconducteurs, et dont la synchronisation est réglée en fonction de la longueur dudit câble et de la structure du circuit d'interface du capteur CCD,
- un panneau de commande permettant notamment de paramétrer le fonctionnement du processeur vidéo en fonction de la température de couleur de l'éclairement de la cible par l'extrémité distale du faisceau de fibres d'éclairage de l'endoscope sur lequel est connectée la tête de caméra, et
- un moniteur vidéo relié au processeur vidéo.

Qu'il soit intégré dans une sonde vidéoendoscopique ou dans une caméra d'endoscopie, le processeur vidéo présente une structure fonctionnelle comprenant :
- des moyens de traitement du signal intégrés dans un circuit plus ou moins complexe, et donc plus ou moins volumineux, désigné sous le terme générique de DSP (Digital Signal Processor), recevant le signal électrique délivré par le capteur CCD et délivrant un signal vidéo de nature analogique ou numérique suivant le type ou les modalités de mise en oeuvre dudit DSP,
- des moyens de synchronisation, intégrés ou associés au DSP, et agissant simultanément sur le capteur CCD et sur les moyens de traitement du signal,
- des moyens d'encodage et d'amplification, intégrés ou associés au DSP, et permettant de transformer le signal vidéo délivré par le DSP en un ou plusieurs signaux vidéo de nature analogique (composite, YC, RGB, ...) et/ou de nature numérique (USB, I.LINK, VGA, ETHERNET, ...),
- des moyens de commande logique du DSP, constitués d'un microcontrôleur intégré ou associé au DSP,
- des moyens d'introduction de commandes gérés par l'utilisateur et consistant généralement en un panneau de touches tactiles directement associé au microcontrôleur, et
- des moyens d'alimentation générant les tensions électriques nécessaires au fonctionnement du capteur CCD et du processeur vidéo.

Le fonctionnement conjoint d'un capteur CCD couleur et du processeur vidéo auquel il est associé résulte essentiellement d'une gestion correcte des déphasages des différentes horloges rapides générées par les moyens de synchronisation du processeur vidéo.

Ces horloges rapides comprennent tout d'abord les signaux de synchronisation "pixel" qui sont transmis au capteur CCD distal qui les utilise d'une part pour synchroniser la lecture des tensions électriques contenues dans les cellules unitaires (dénommées pixels) de la couche photosensible du capteur, et d'autre part pour extraire de ces tensions unitaires les informations significatives qui constituent, après intégration, le signal électrique transmis au processeur vidéo. Ces horloges rapides comprennent également les signaux de synchronisation qui sont transmis au processeur vidéo qui les utilise pour synchroniser l'échantillonnage du signal électrique généré par le capteur CCD.

Le fonctionnement correct du processeur exige impérativement que son horloge d'échantillonnage soit parfaitement en phase avec le signal électrique incident provenant du capteur CCD. Or, le déport du capteur CCD couleur dans la tête d'une caméra endoscopique ou dans l'extrémité distale d'une sonde vidéoendoscopique introduit inévitablement, du fait de la longueur des liaisons électriques reliant le capteur au processeur vidéo et des caractéristiques du circuit d'interface éventuellement associé au capteur, un déphasage rédhibitoire au niveau du processeur entre l'horloge d'échantillonnage et le signal électrique incident. Ce déphasage résulte du cumul du délai de transmission vers le capteur CCD des signaux de synchronisation pixel générés par le processeur vidéo, du délai de transmission vers le processeur vidéo du signal électrique généré par le capteur CCD, et des déphasages introduits par le circuit d'interface du capteur CCD. Généralement, pour remédier à un tel dysfonctionnement, on retarde soit l'horloge d'échantillonnage, soit l'horloge de synchronisation pixel, et ce de manière à compenser le déphasage global évoqué ci-dessus. Les modalités de mise en oeuvre de l'un ou de l'autre de ces retards et les problèmes de connectique qui en découlent varient en fonction du mode d'intégration des moyens de synchronisation et de traitement du signal qui peuvent, selon l'architecture retenue, soit être externes à la caméra d'endoscopie ou à la sonde vidéoendoscopique, soit en faire partie intégrante.

Traditionnellement, le processeur vidéo d'une caméra d'endoscopie ou d'une sonde vidéoendoscopique est logé dans un coffret externe sur lequel vient se raccorder un connecteur multibroches constituant l'extrémité proximale du tube ombilical de la caméra ou de la sonde vidéoendoscopique. Dans une telle architecture, le panneau de touches de commande est généralement intégré sur la face avant du coffret externe, et le moniteur vidéo est directement raccordé au coffret.

Il ressort des considérations évoquées ci-avant que le raccordement d'une caméra d'endoscopie ou d'une sonde vidéoendoscopique à un processeur vidéo logé dans un coffret externe, entraîne des problèmes d'adaptation dus à la nécessité de compenser les retards de synchronisation induits par le circuit d'interface du capteur CCD de la sonde ou de la caméra et par le câble électrique reliant le circuit d'interface au processeur vidéo auquel le capteur CCD est associé. Si le coffret externe est destiné à toujours être associé à un même modèle de caméra endoscopique, on se trouve en présence d'un problème d'interchangeabilité imposant au câble ombilical des caméras d'avoir toujours exactement la même longueur. Si le coffret externe est destiné à être associé, par exemple, à une gamme de sondes vidéoendoscopiques présentant différentes longueurs (comme cela est décrit dans le brevet US 4 539 568), on se trouve confronté à un problème de compatibilité nécessitant l'intégration dans le boîtier de connexion ou dans la poignée de commande de la sonde d'un dispositif spécifique de retard agissant sur les horloges rapides générées par le processeur vidéo et transmises au capteur CCD distal.

Quoiqu'il en soit, la mise en oeuvre d'un processeur vidéo externe présente un autre inconvénient technique concernant les risques de parasitage du signal électrique généré par le capteur CCD et transmis au processeur vidéo. En effet, le transport du signal électrique généré par le capteur CCD s'avère délicat en raison tant de son faible rapport signal/bruit intrinsèque, que de sa large bande passante et de sa faible puissance nécessitant une liaison à impédance élevée qui ne contribue pas à offrir une bonne immunité aux parasites. La qualité d'un tel signal électrique risque en outre d'être altérée par le vieillissement ou toute autre défaillance des contacts du système de connexion multibroches permettant de relier l'extrémité proximale du tube ombilical de la caméra ou de la sonde au coffret externe logeant le processeur vidéo.

Un moyen de palier à tout ou partie des inconvénients évoqués ci-avant consisterait à intégrer le processeur vidéo au plus près du capteur CCD distal, c'est-à-dire dans le cas d'une caméra endoscopique, directement dans la tête de la caméra, et dans le cas d'une sonde vidéoendoscopique, dans la poignée de commande, et dans ce dernier cas, à relier électriquement le capteur CCD au processeur vidéo à l'aide d'une liaison ne présentant aucun risque de rupture de continuité. L'encombrement intrinsèque d'un processeur vidéo s'avère malheureusement incompatible avec le volume disponible tant dans une tête de caméra que dans une poignée de sonde de faibles dimensions. Les seules réalisations connues en la matière concernent l'intégration du processeur vidéo soit dans une poignée de sonde vidéoendoscopique à vocation industrielle disposant d'un moniteur vidéo intégré (brevet FR 2 785 132), soit dans un boîtier de connexion fixement solidaire de l'extrémité proximale du câble ombilical d'une sonde à vocation industrielle (brevet US 5 702 345).

US 2002/0171733 décrit un videoendoscope ayant les caractéristiques du préambule de la revendication 1. Spécifiquement, US 2002/0171733 décrit l'électronique d'un vidéo-endoscope, comprenant un circuit de prétraitement vidéo et un circuit auxiliaire, reliés par un connecteur. Le circuit de prétraitement, logé dans le connecteur, comprend un micro-contrôleur, un circuit d' échantillonnage et un DSP vidéo fournissant des signaux vidéo bruts au circuit auxiliaire.

La présente invention a pour but de supprimer ces inconvénients.

L'invention est définie par les revendications.

Un mode de réalisation préféré de l'invention sera décrit ci-après, à titre d'exemple non limitatif, avec référence aux dessins annexés dans lesquels :
La figure 1 représente sous la structure fonctionnelle d'un processeur vidéo pour endoscopie, selon la présente invention ;
La figure 2 représente une variante, ne faisant pas partie de l'invention, de la structure fonctionnelle d'un processeur vidéo pour endoscopie, montrée sur la figure 1 ;
La figure 3 illustre un exemple de mise en oeuvre dans une sonde vidéoendoscopique, du processeur vidéo représenté sur la figure 1 ;
La figure 4 illustre un exemple de mise en oeuvre dans une caméra d'endoscopie, du processeur vidéo représenté sur la figure 1 ;
La figure 5 illustre un exemple de mise en oeuvre dans une caméra d'endoscopie, du processeur vidéo représenté sur la figure 2.

La présente invention vise à intégrer un processeur vidéo dans une sonde vidéoendoscopique ou dans une caméra d'endoscopie, de faibles dimensions.

Cet objectif est atteint en séparant physiquement sur deux cartes reliées l'une à l'autre par une liaison électrique, les fonctions de traitement du signal proprement dites, des fonctions auxiliaires d'un processeur vidéo, la carte regroupant les fonctions de traitement du signal présentant un encombrement minimum pour pouvoir être intégrée aussi bien dans une tête de caméra d'endoscopie que dans la poignée de commande d'une sonde vidéoendoscopique. A cet effet, la présente invention propose

La première de ces deux cartes est reliée au capteur CCD par une liaison électrique ne présentant aucun risque de rupture de continuité. Elle est destinée à être logée au plus près du capteur CCD, et donc de préférence soit dans la poignée d'une sonde vidéoendoscopique, soit dans la tête d'une caméra d'endoscopie. A cet effet, en vue de présenter un encombrement minimum, elle ne supporte que les moyens électroniques strictement nécessaires pour assurer les fonctions suivantes :
- une fonction de traitement du signal électrique délivré par le capteur CCD élaborant deux signaux bruts de luminance (Y) et chrominance (C) présentant des puissances suffisantes pour pouvoir être véhiculés par des liaisons électriques à basse impédance, offrant une bonne immunité aux parasites,
- une fonction de synchronisation du capteur CCD et de la fonction de traitement du signal, et
- éventuellement, une fonction de correction de la synchronisation du capteur CCD en fonction de la distance de son éventuel déport et des déphasages introduits par son association éventuelle à un circuit d'interface.

La seconde de ces deux cartes, logée de préférence dans un boîtier de connexion fixement solidaire de l'extrémité proximale du tube ombilical d'une sonde vidéoendoscopique ou d'une caméra d'endoscopie, supporte les moyens électroniques permettant d'assurer les fonctions suivantes :
- une fonction de commande logique de la fonction de traitement de la première carte,
- une fonction de contrôle de la fonction de commande par des touches intégrées de préférence sur le boîtier de connexion, et/ou par une liaison logique connectée à des moyens informatiques externes,
- une fonction de traitement des signaux bruts Y et C délivrés par la première carte et d'élaboration d'un ou plusieurs signaux vidéo utiles de nature analogique et/ou numérique, et
- une fonction d'alimentation en diverses tensions électriques continues nécessaires au fonctionnement des deux cartes ainsi que du capteur CCD.

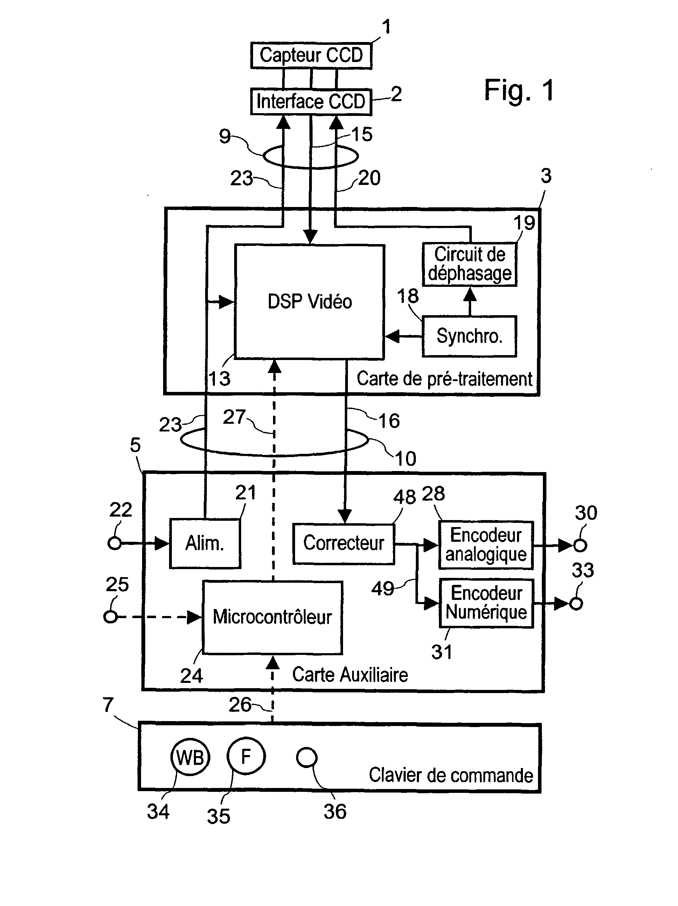

La figure 1 représente l'architecture électronique d'un processeur vidéo pour endoscopie, selon l'invention. Ce processeur vidéo est de façon avantageuse, conçu pour se connecter à un capteur CCD 1 du type couleur à transfert de ligne, par l'intermédiaire d'un circuit d'interface 2.

Sur cette figure, le processeur vidéo comprend une carte de pré-traitement 3 connectée au capteur CCD 1 par l'intermédiaire du circuit d'interface 2 et une carte auxiliaire 5 connectée à la carte de pré-traitement 3. Le circuit d'interface 2 est destiné à remettre en forme des signaux de synchronisation transmis par la carte de pré-traitement par l'intermédiaire d'une liaison électrique 20 et à améliorer l'adaptation d'impédance du signal électrique délivré par le capteur CCD, le signal résultant étant transmis à la carte de pré-traitement par l'intermédiaire d'une liaison électrique 15 à haute impédance.

La carte de pré-traitement 3 supporte un générateur 18 d'horloges de synchronisation, et un processeur numérique 13 de traitement de signal vidéo, synchronisé par le générateur 18. Le processeur numérique reçoit par la liaison électrique 15, le signal électrique généré par le capteur CCD couleur 1 et adapté par le circuit d'interface 2, et délivre sur une liaison 16 un signal vidéo brut analogique non directement visualisable sur un écran vidéo, mais présentant une puissance suffisante pour pouvoir être transmis par une liaison à basse impédance présentant de ce fait une bonne immunité aux parasites.

Le processeur 13 de traitement de signal est avantageusement constitué par un DSP (Digital Signal Processor) vidéo, commandé par un signal numérique série, par exemple de type TTL provenant de la carte auxiliaire par une liaison 27. Le générateur 18 synchronise également le capteur CCD 1 par l'intermédiaire de la liaison 20. Le processeur 13 est de préférence un DSP non programmable et simplement paramétrable, dédié au capteur vidéo choisi, de manière à présenter un encombrement minimum afin de pouvoir par exemple être facilement intégrable dans la poignée d'une sonde vidéoendoscopique.

La carte 3 de pré-traitement est reliée à la carte 5 auxiliaire par un câble multiconducteurs 10 comprenant les liaisons 23 d'alimentation électrique de la carte 3 et du capteur CCD 1, la liaison de commande 27 du processeur DSP 13, et la liaison 16 de transmission du signal vidéo analogique brut délivré par le processeur DSP 13.

La carte 5 auxiliaire supporte un dispositif d'alimentation électrique 21, un processeur 24, par exemple de type microcontrôleur, un circuit de correction analogique 48 et des encodeurs 28, 31 délivrant respectivement des signaux vidéo utiles 30, 33, c'est-à-dire directement visualisables sur un écran vidéo. Le dispositif d'alimentation 21 qui est destiné à être relié à une source d'énergie électrique externe 22, élabore les différentes tensions continues d'alimentation, nécessaires au fonctionnement des cartes 3, 5 et du capteur CCD couleur 1. Le microcontrôleur 24, commandé par une liaison 25, par exemple de type RS232, et par une liaison 26, par exemple de type TTL, provenant d'un clavier de commande 7, de préférence à touches sensitives, délivre des signaux numériques série 27, servant à commander le processeur DSP 13.

La liaison 25 est destinée à être raccordée à un ordinateur (non représenté) dont le clavier peut être utilisé pour introduire des commandes, et l'écran d'affichage pour afficher le signal vidéo numérique utile 33.

Le circuit de correction 48 reçoit par la liaison 16 le signal vidéo analogique brut délivré par le processeur DSP 13, à partir duquel il génère un signal vidéo utile qui est transmis par la liaison 49 aux encodeurs 28, 31 constituant l'interface de sortie vidéo du processeur DSP 13.

Le signal vidéo analogique brut délivré par le processeur DSP 13 comprend une composante luminance Y et une composante chrominance C, ces composantes étant dites brutes parce que non en phases et bruitées notamment par des résidus d'horloge de synchronisation (non filtrées), mais présentant une puissance suffisante pour pouvoir être transmis par une liaison à basse impédance présentant de ce fait une bonne immunité aux parasites.

Le circuit de correction analogique 48 effectue un filtrage de type passe-bande, et une mise en phase des composantes Y et C du signal vidéo analogique brut, et transmet le signal vidéo utile résultant par la liaison 49 aux dispositifs d'encodage 28, 31.

Le dispositif d'encodage analogique 28 délivre un signal vidéo analogique utile 30 de type vidéo composite, tandis que le dispositif d'encodage numérique 31 délivre un signal vidéo numérique utile 33, par exemple de type USB2, les signaux vidéo 30, 33 étant directement visualisables sur un écran vidéo.

Le clavier de commande 7 à touches sensitives est un clavier simplifié de faible encombrement comprenant une touche 34 permettant de paramétrer automatiquement le fonctionnement du processeur DSP 13 en fonction de la température de couleur de la lumière délivrée par l'extrémité distale de la sonde vidéoendoscopique ou de l'endoscope associé à une caméra d'endoscopie, une touche 35 permettant d'activer une fonction de paramétrage du processeur DSP 13, cette fonction étant préprogrammée dans le microcontrôleur 24, et une diode 36 signalant l'activation de cette fonction.

De cette manière, la sonde vidéoendoscopique ou la caméra vidéo d'endoscopie intégrant le processeur vidéo selon l'invention s'adapte automatiquement à la température de couleur du générateur de lumière auquel est connecté le faisceau de fibres d'éclairage de la sonde vidéoendoscopique ou de l'endoscope, et donc in fine, en fonction du type de lampe d'éclairage (halogène, vapeur de mercure, xénon, ...) du générateur de lumière. Ces derniers peuvent donc être associés à n'importe quel générateur de lumière.

La mise en oeuvre optionnelle d'un ordinateur portable de type PC équipé d'une entrée USB2 connectée sur la sortie 33 de la carte 5 auxiliaire du processeur vidéo et d'un port RS 232 connecté sur l'entrée 25 du microcontrôleur 24 de la carte auxiliaire, permet à l'utilisateur d'exploiter directement le processeur vidéo à partir de moyens de nature informatique et de bénéficier de toutes les potentialités de paramétrage et de réglage du DSP vidéo 13.

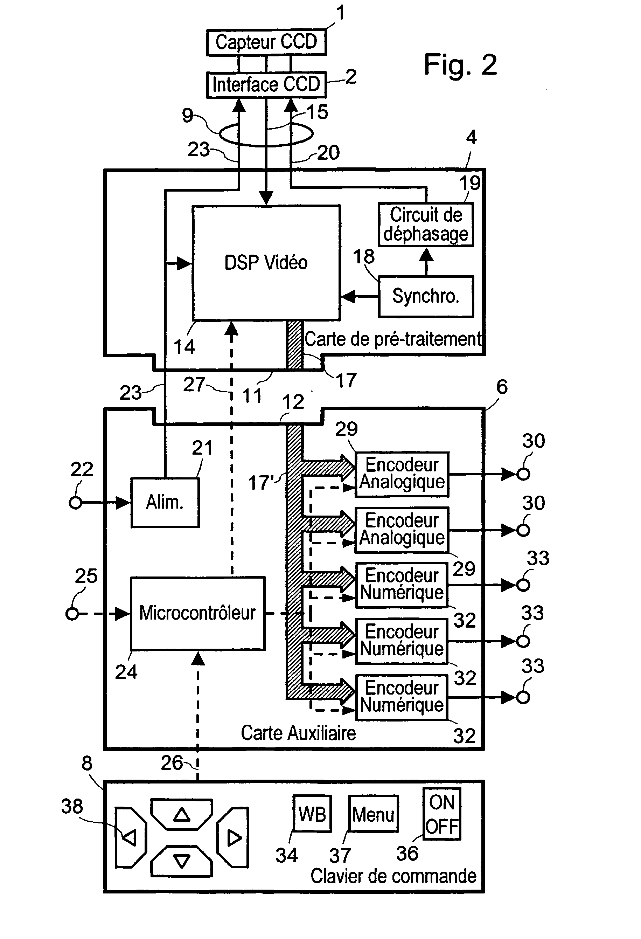

La figure 2 illustre une variante, ne faisant pas partie de l'invention, du processeur vidéo représenté sur la figure 1. Sur cette figure, le capteur CCD couleur 1 associé au circuit d'interface 2, est couplé à un processeur vidéo dont les éléments sont répartis sur une carte de pré-traitement 4 et une carte auxiliaire 6. Le circuit d'interface 2 est relié à la carte 4 de pré-traitement par un câble multiconducteurs 9 comprenant les liaisons 23 d'alimentation électrique du capteur CCD, les liaisons 20 de synchronisation du capteur CCD, et la liaison 15 de transmission du signal délivré par le capteur CCD.

La carte 4 de pré-traitement supporte un générateur d'horloges 18 de synchronisation, un circuit de déphasage 19 et un processeur numérique de traitement de signal vidéo (DSP) 14, commandé par un signal numérique série, et directement synchronisé par le générateur d'horloges 18. Le processeur DSP vidéo 14 reçoit le signal électrique 15 délivré par le capteur CCD 1 et génère un signal vidéo brut de nature numérique transmis à la carte auxiliaire 6 par une liaison 17. Les horloges de synchronisation "pixel" du capteur CCD sont délivrées par le générateur d'horloges 18, puis retardées par le circuit de déphasage 19 avant d'être transmises par la liaison électrique 20 au capteur CCD, et ce de façon à compenser les retards de propagation introduits par les liaisons électriques 15 et 20, ainsi que les déphasages introduits par le circuit d'interface 2.

Le processeur 14 est également de préférence un DSP non programmable et simplement paramétrable, dédié au capteur vidéo choisi, de manière à présenter un encombrement minimum afin de pouvoir par exemple être facilement intégrable dans la poignée d'une sonde vidéoendoscopique.

La carte auxiliaire 6 supporte le dispositif d'alimentation électrique 21, le microcontrôleur 24, et plusieurs encodeurs 29, 32 recevant par la liaison 17 le signal vidéo brut numérique et délivrant respectivement des signaux vidéo utiles 30, 33. Le dispositif d'alimentation 21, relié à une source d'énergie électrique externe 22, élabore les différentes tensions continues d'alimentation, nécessaires au fonctionnement des cartes 4, 6 et du capteur CCD couleur 1. Le microcontrôleur 24, commandé par une liaison 25, et par la liaison 26 provenant d'un clavier de commande 8 à touches sensitives, délivre sur la liaison 27 les signaux numériques série servant à commander le processeur DSP 14. A titre d'exemple, la liaison 25 est de type RS 232 et les liaisons 26 et 27 sont de type TTL.

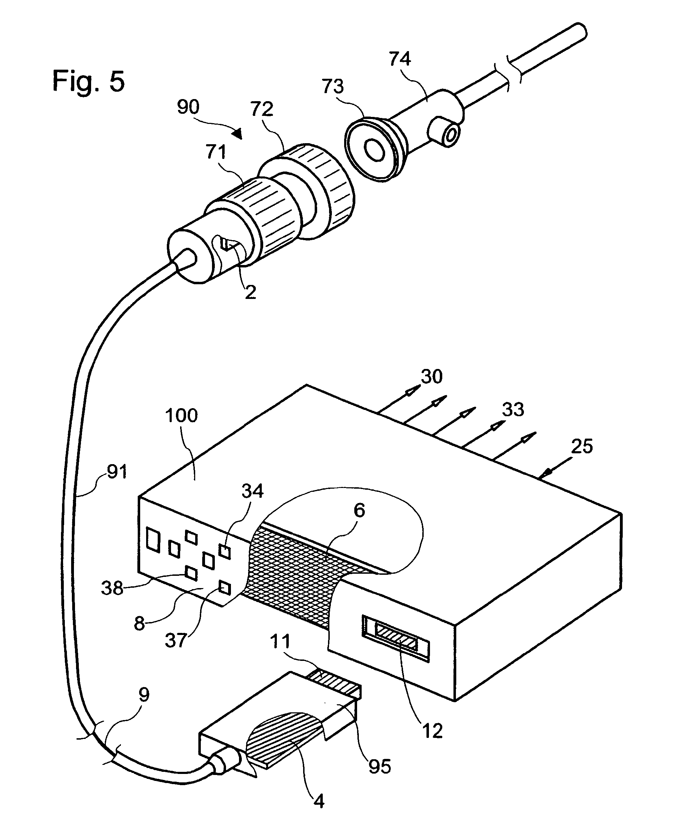

Compte tenu de son encombrement intrinsèque, la carte auxiliaire 6 est logée dans un coffret externe. Compte tenu de la complexité intrinsèque de son interface de sortie, la carte 4 de pré-traitement est logée dans un boîtier de connexion solidaire de l'extrémité proximale d'un câble ombilical 9 (figure 5) directement relié au coffret externe par un dispositif de connexion 11, 12.

Le signal vidéo numérique délivré par le processeur DSP 14 sur la liaison 17 comprend de préférence deux signaux numériques de huit bits chacun correspondant aux composantes brutes de chrominance C et de luminance Y du signal vidéo, ces composantes étant dites brutes parce que non en phase et bruitées notamment par des résidus d'horloge de synchronisation. Suivant le type de processeur DSP, la liaison 17 transmettant les composantes numériques Y et C, est constituée soit d'un bus 16 bits, soit d'un bus 8 bits dans lequel les composantes Y et C sont multiplexées.

Les encodeurs 29, 32 comprennent un ou plusieurs encodeurs numériques/analogiques qui délivrent respectivement des signaux vidéo analogiques utiles 30 par exemple de type composite, YC et RGB, et un ou plusieurs encodeurs numériques/numériques 32 qui délivrent respectivement des signaux vidéo numériques utiles 33 par exemple de type USB2 et VGA.

Les encodeurs 29 et 32 sont réalisés avec des composants du commerce intégrant des fonctions de filtrage anti-aliasing et de mise en phase nécessaires aux corrections du signal vidéo numérique brut de type YC produit par le processeur DSP 14.

Le clavier de commande 8 à touches tactiles comprend une touche 34 permettant de paramétrer automatiquement le fonctionnement du processeur DSP 14 en fonction de la température de couleur de la lumière délivrée par l'extrémité distale de la sonde vidéoendoscopique ou de l'endoscope associé à la caméra d'endoscopie, une touche 37 permettant d'entrer dans le menu de réglage du processeur DSP 14, et quatre touches de navigation 38 permettant de sélectionner et de régler les différentes fonctions du menu.

La mise en oeuvre optionnelle d'un ordinateur portable de type PC équipé d'une entrée USB2 connectée sur la sortie 33 de type USB2 de la carte 6 auxiliaire et d'un port RS 232 connecté sur l'entrée 25 du microcontrôleur 24 permet à l'utilisateur d'exploiter le processeur vidéo selon l'invention, directement à partir de moyens de nature informatique.

Les architectures selon l'invention, illustrées sur les figures 1 et 2, permettent de réduire le plus possible le nombre et la taille des composants à placer au plus près du capteur vidéo (sur la carte de pré-traitement), les fonctions nécessaires à la génération d'un signal vidéo utile qui ne sont pas réalisées par la carte de pré-traitement étant déportées à distance sur la carte auxiliaire.

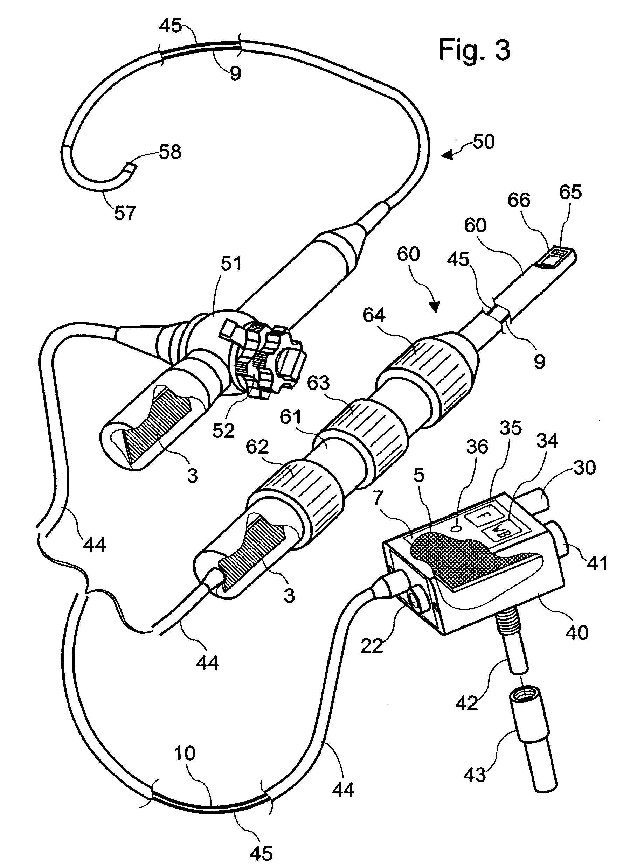

La figure 3 illustre les architectures d'une sonde vidéoendoscopique souple 50 et d'une sonde vidéoendoscopique rigide 60, mettant en oeuvre le processeur vidéo décrit ci-avant en référence à la figure 1.
Dans ces architectures, la carte 3 de pré-traitement est logée dans les extrémités proximales des poignées de commande 51 et 61 des sondes 50 et 60, ces poignées étant solidaires de l'extrémité distale d'un câble ombilical 44 dont l'extrémité proximale est solidaire d'un boîtier de connexion 40 logeant la carte auxiliaire 5 et supportant le clavier de commande simplifié 7.

La sonde vidéoendoscopique souple 50 comprend les éléments suivants :
- un embout distal 58 solidaire d'un béquillage 57 et logeant un objectif, le capteur CCD 1, le circuit d'interface 2, et l'extrémité distale d'un faisceau de fibres d'éclairage 45 ;
- un tube d'inspection souple dont l'extrémité distale est solidaire du béquillage 57 et qui loge des câbles de commande de béquillage, le faisceau de fibres d'éclairage 45 et le câble multiconducteurs 9 reliant électriquement le circuit d'interface 2 du capteur CCD 1 à la carte de pré-traitement 3 ;
- une poignée de commande 51 solidaire de l'extrémité proximale du tube d'inspection souple et de l'extrémité distale du câble ombilical 44, la poignée logeant la carte de pré-traitement 3 et supportant des moyens d'actionnement 52 permettant à l'utilisateur d'agir sur les câbles de commande du béquillage 57 ; et
- le câble ombilical 44 servant de logement au faisceau de fibres d'éclairage 45 et au câble multiconducteurs 10 reliant électriquement la carte de pré-traitement 3 à la carte auxiliaire 5.

La sonde vidéoendoscopique rigide 60 est une sonde à visée déviée comportant avantageusement des commandes de rotation de l'axe de visée, de variation de l'angle de visée, et de réglage de mise au point. Cette sonde vidéoendoscopique, qui met en oeuvre des dispositifs mécaniques de commande identiques aux dispositifs intégrés dans les endoscopes multi-fonctions décrits dans le brevet FR 2 832 516 déposé par la Demanderesse, résulte de l'association fonctionnelle des éléments décrits ci-après.

Une extrémité distale disposant d'une fenêtre de vision latérale 66 et d'une fenêtre d'illumination latérale 65 logeant l'extrémité distale du faisceau de fibres d'éclairage 45, et logeant un dispositif optoélectronique comprenant un prisme déviateur distal, un objectif et le capteur CCD 1 associé à son circuit d'interface 2.

Un tube d'inspection rigide logeant un tube de commande de mise au point dont l'extrémité distale est solidaire du capteur CCD, le câble multiconducteurs 9 logé dans le tube de commande de mise au point et reliant électriquement le circuit d'interface 2 du capteur CCD 1 à la carte de pré-traitement 3, un tube de commande de visée logé de façon coulissante autour du tube de commande de mise au point et dont l'extrémité distale commande le basculement du prisme déviateur distal, et un faisceau de fibres d'éclairage 45 dont les fibres sont réparties dans le volume annulaire compris entre le tube de commande de visée et le tube externe de la sonde 60.

Une poignée de commande 61 solidaire de l'extrémité distale du câble ombilical 44, logeant la carte de pré-traitement 3, et supportant une bague distale 64 solidaire de l'extrémité proximale du tube d'inspection rigide et permettant de commander la rotation du tube autour de son axe, une bague centrale 63 permettant de commander les déplacements en translation du tube de commande de visée et donc le basculement du prisme déviateur distal, et une bague proximale 62 permettant de commander les déplacements en translation du tube de commande de mise au point et donc les déplacements en translation du capteur CCD.

Un câble ombilical 44 logeant le faisceau de fibres d'éclairage 45 et le câble multiconducteurs 10 reliant électriquement la carte de pré-traitement 3 à la carte auxiliaire 5.

Le boîtier de connexion 40 solidaire de l'extrémité proximale du câble ombilical 44 dispose d'un embout d'éclairage 42 logeant l'extrémité proximale du faisceau de fibres d'éclairage 45 et susceptible de recevoir différents types d'adaptateurs mécaniques 43 permettant de connecter le boîtier 40 à différents types de générateurs de lumière. Le boîtier de connexion 40 loge la carte auxiliaire 5 qui se trouve ainsi associée au clavier de commande simplifié 7, conformément à la description ci-avant de la figure 1. Le boîtier de connexion 40 comprend en outre :
- une embase de connexion d'alimentation 22 destinée à être raccordée à une source d'énergie électrique externe, de préférence un générateur autonome délivrant une tension électrique continue banalisée, par exemple de 12 volts,
- une embase de connexion vidéo 30 destinée à être raccordée à un moniteur vidéo et délivrant un signal vidéo analogique préférentiellement de type composite, et
- une embase de connexion multibroches de type informatique 41 destinée à être raccordée soit à un système numérique dédié de traitement d'images, soit à un ordinateur standard de type PC, cette embase regroupant une liaison RS232 associée soit à une connexion vidéo analogique par exemple de type composite, soit à une connexion vidéo numérique par exemple de type USB2.

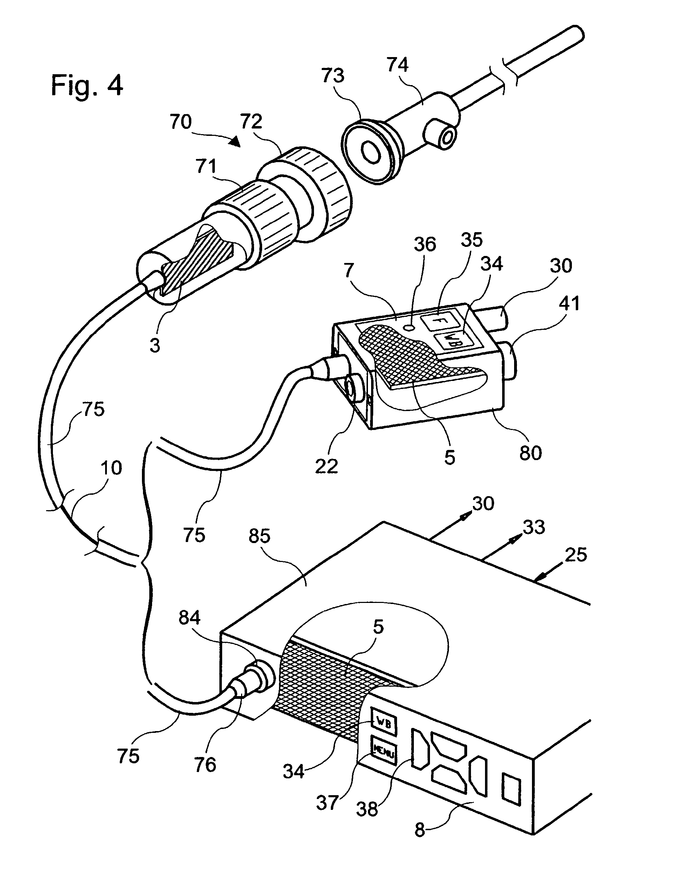

La figure 4 illustre les architectures de deux caméras d'endoscopie 70, 80 et 70, 85 mettant en oeuvre le processeur vidéo décrit ci-avant en référence à la figure 1, et associant la carte 3 de pré-traitement et la carte auxiliaire 5. Dans ces architectures, la carte 3 de pré-traitement est logée dans une tête de caméra 70 solidaire de l'extrémité distale d'un câble ombilical 75 dont l'extrémité proximale est soit solidaire d'un boîtier de connexion 80 logeant la carte auxiliaire 5 et supportant le clavier de commande simplifié 7, soit connectable à un coffret externe 85 logeant la carte 5 auxiliaire et supportant le clavier de commande 8 décrit ci-avant en référence à la figure 2.

La tête de caméra 70 résulte de l'association fonctionnelle des éléments suivants :
- un objectif dont la mise au point est commandée par la rotation d'une bague de réglage externe 71,
- un dispositif 72 solidaire de la partie distale de l'objectif et permettant de verrouiller mécaniquement la tête de caméra sur la bonnette proximale 73 d'un endoscope 74,
- le capteur CCD 1 sur la surface photosensible duquel se forme l'image délivrée par l'objectif,
- la carte de pré-traitement 3 directement associée au capteur CCD, et
- un câble ombilical 75 logeant le câble multiconducteurs 10 reliant électriquement la carte 3 de pré-traitement à la carte auxiliaire 5.

Le boîtier de connexion 80 solidaire de l'extrémité proximale du câble ombilical 75 loge la carte auxiliaire 5 qui se trouve associée, conformément au texte relatif à la figure 1, à un clavier de commande simplifié 7 disposant des deux touches tactiles 34, 35 et du voyant lumineux 36. Le boîtier de connexion dispose d'un interface comportant les embases de connexion 22, 30 et 41 décrites ci-avant en référence à la figure 3.

Le coffret 85 dispose d'une embase de connexion 84 électriquement solidaire de la carte auxiliaire 5 logée dans ledit coffret, cette embase étant destinée à être raccordée au connecteur 76 solidaire de l'extrémité proximale du câble ombilical 75 et transmettant les signaux électriques véhiculés par le câble multiconducteurs 10 logé dans le câble ombilical 75 et assurant la liaison entre la carte 3 de pré-traitement et la carte 5 auxiliaire. La carte auxiliaire 5 est associée au clavier de commande 8 décrit ci-avant en référence à la figure 2.

Il est à noter que les sondes 50, 60 montrées sur la figure 3 peuvent alternativement être connectées, en remplacement du boîtier de connexion 40, à un boîtier tel que le boîtier 85 décrit en référence à la figure 4.

La figure 5 illustre l'architecture d'une caméra d'endoscopie 90 mettant en oeuvre le processeur vidéo décrit ci-avant en référence à la figure 2, associant la carte 4 de pré-traitement et la carte auxiliaire 6. La carte 4 de pré-traitement est logée dans un boîtier de connexion 95 solidaire de l'extrémité proximale d'un câble ombilical 91 de la tête de caméra 90 et connectable à un coffret externe 100 logeant la carte auxiliaire 6 et supportant le clavier de commande 8.

La tête de caméra 90 résulte de l'association fonctionnelle des éléments suivants :
- un objectif dont la mise au point est commandée par la rotation d'une bague de réglage externe 71,
- un dispositif mécanique 72 solidaire de la partie distale de l'objectif et permettant de verrouiller la tête de caméra sur la bonnette proximale 73 d'un endoscope 74,
- le capteur CCD 1 sur la surface photosensible duquel se forme l'image délivrée par l'objectif,
- le circuit d'interface 2 directement associé au capteur CCD, et
- le câble ombilical 91 logeant le câble multiconducteurs 9 reliant électriquement le circuit d'interface 2 du capteur CCD à la carte 4 de pré-traitement.

Le boîtier de connexion 95 dispose du connecteur multibroches 11 électriquement solidaire de la carte de pré-traitement 4 logée dans le boîtier 95, tandis que le coffret 100 dispose de l'embase de connexion multibroches 12 électriquement solidaire de la carte auxiliaire 6 logée dans ledit coffret, cette embase étant destinée à recevoir le connecteur 11 du boîtier de connexion 95. Conformément à la description ci-avant de la figure 2, ne faisant pas partie de l'invention, la carte auxiliaire 6 est associée au clavier de commande 8. Le coffret 100 dispose par ailleurs d'embases de connexions distribuant un ou plusieurs signaux vidéo 30 de nature analogique, un ou plusieurs signaux vidéo 33 de nature numérique, et une voie de commande 25 de type RS 232.

Il est à noter que l'architecture illustrée sur la figure 5 peut aussi être adaptée aux sondes vidéoendoscopiques montrées sur la figure 3.

## Revendications

1. Vidéoendoscope comprenant au moins
- une sonde (50, 60, 70) comprenant un capteur vidéo (1) couleur,
- une poignée de commande (51, 61) relié à une extrémité de la sonde, et
- un processeur vidéo comprenant :
o un circuit de pré-traitement de signal, localisé sur une carte de pré-traitement (3, 4), et déportable à distance du capteur vidéo par une première liaison électrique (15), le circuit de pré-traitement comprenant
■ des premiers moyens (13, 14) de traitement de signal pour générer à partir des signaux d'image délivrés par le capteur vidéo, des signaux vidéo bruts (16, 17) comportant un signal de luminance et un signal de chrominance, et
■ des moyens de synchronisation (18, 19) pour synchroniser le capteur vidéo et les premiers moyens de traitement de signal ; et
o un circuit auxiliaire, localisé sur une carte de auxiliaire (5, 6), et comprenant des seconds moyens de traitement de signal, reliés aux premiers moyens de traitement de signal, et comprenant des moyens (48, 28, 29, 31, 32) pour générer à partir des signaux vidéo bruts de luminance et de chrominance au moins un signal vidéo utile (30, 33) conforme à un standard international.
**caractérisé en ce que** le vidéoendoscope comprend en outre un boîtier de connexion (40, 80) ou un coffret externe (85) relié à la poignée de commande,
**en ce que**
- la carte de pré-traitement (3, 4) est disposée dans la poignée de commande (51, 61),
- les signaux vidéo bruts de chrominance et de luminance sont déphasés et bruités par des résidus de synchronisation,
- la première liaison électrique (15) est à haute impédance et dépourvue de dispositif de connexion intermédiaire,
- la carte auxiliaire (5, 6) est déportée à distance de la carte de pré-traitement (3, 4), et disposée dans le boîtier de connexion (40, 80) ou dans le coffret externe (85), **en ce que** le circuit auxiliaire (5, 6) comprend des moyens de commande (24) de type microcontrôleur numérique pour paramétrer lesdits premiers moyens de traitement (13, 14) de signal, et **en ce que**
- un câble ombilical (44, 75) sert de logement à un câble multiconducteur (10) à basse impédance, offrant une bonne immunité aux parasites, reliant électriquement la carte de pré-traitement (3, 4) à la carte auxiliaire (5, 6).

2. Vidéoendoscope selon la revendication 1,
**caractérisé en ce que** les signaux vidéo bruts de chrominance et de luminance sont de nature analogique, les seconds moyens de traitement de signal comprenant un circuit de correction (48) pour mettre en phase et filtrer les signaux vidéo bruts de luminance et de chrominance, et au moins un circuit d'encodage (28, 31) pour générer à partir des signaux vidéo bruts mis en phase et filtrés (49) un signal vidéo utile analogique ou numérique (30, 33) conforme à un standard international.

3. Vidéoendoscope selon la revendication 1,
**caractérisé en ce que** les signaux vidéo bruts de chrominance et de luminance sont de nature numérique, les seconds moyens de traitement de signal comprenant au moins un circuit de correction et d'encodage (29, 32) pour générer à partir des signaux vidéo bruts de luminance et de chrominance un signal vidéo utile analogique ou numérique (30, 33) conforme à un standard international.

4. Vidéoendoscope selon l'une des revendications 1 à 3,
**caractérisé en ce que** le capteur vidéo (1) est un capteur CCD couleur du type à transfert de ligne.

5. Vidéoendoscope selon l'une des revendications 1 à 4,
**caractérisé en ce que** le circuit de pré-traitement (3, 4) de signal est solidaire du capteur vidéo (1).

6. Vidéoendoscope selon l'une des revendications 1 à 3,
**caractérisé en ce que** le circuit de pré-traitement (3, 4) de signal est connecté par un câble électrique multiconducteurs (15) au capteur vidéo (1) qui se trouve déporté à distance du circuit de pré-traitement, et comprend des moyens de déphasage (19) pour retarder les signaux de synchronisation (20) transmis au capteur vidéo (1)., afin de compenser des délais de transmission introduits par le câble électrique (15).

7. Vidéoendoscope selon l'une des revendications 1 à 6,
**caractérisé en ce que** le circuit de pré-traitement (3, 4) de signal est relié directement au circuit auxiliaire (5, 6) par un câble électrique (10) multiconducteurs, sans dispositif de connexion intermédiaire.

8. Vidéoendoscope selon l'une des revendications 1 à 5,
**caractérisé en ce que** le circuit de pré-traitement (3, 4) de signal est relié au circuit auxiliaire (5, 6) par une liaison électrique (10) associée à un dispositif de connexion (76, 84, 11, 12) intermédiaire.

9. Vidéoendoscope selon la revendication 8,
**caractérisé en ce que** les moyens de commande (24) sont reliés à des moyens (7, 8) d'introduction de commandes opérateur comprenant une touche de commande (34) pour déclencher le paramétrage des premiers moyens de traitement (13, 14) de signal en fonction d'une température de couleur d'illumination captée par le capteur vidéo (1).

10. Vidéoendoscope selon l'une des revendications 8 à 9,
**caractérisé en ce que** le circuit auxiliaire (5, 6) comprend une interface logique (25) permettant de relier les moyens de commande (24) à un ordinateur comportant un écran sur lequel le signal vidéo utile (30, 33) peut être visualisé, et un clavier permettant d'introduire des commandes de paramétrage des premiers moyens de traitement (13, 14) de signal.

11. Vidéoendoscope selon l'une des revendications 1 à 10,
**caractérisé en ce que** le circuit auxiliaire (5, 6) comprend un circuit d'alimentation électrique (21) pour générer à partir d'une tension continue des tensions nécessaires à L'alimentation du capteur vidéo (1), du circuit de prétraitement (3, 4) et du circuit auxiliaire (5, 6).

12. Vidéoendoscope selon l'une quelconque des revendications 1 à 11,
**caractérisé en ce que** le vidéoendoscope est une sonde vidéoendoscopique comprenant :
- un tube d'inspection (50, 60) dont l'extrémité distale est solidaire d'un embout distal (58),
- la poignée de commande (51, 61) solidaire de l'extrémité proximale du tube d'inspection,
- un câble ombilical (44) dont l'extrémité distale est solidaire de la poignée de commande,
- un dispositif de connexion (40, 76) solidaire de l'extrémité proximale du câble ombilical, et destiné à être raccordé à un générateur de lumière, et
- un faisceau de fibres d'éclairage (45) logé dans le câble ombilical, dans la poignée de commande, et dans le tube d'inspection, l'extrémité distale du faisceau étant logée dans l'embout distal pour illuminer une cible lorsque l'extrémité proximal du faisceau est raccordée à un générateur de lumière,
sonde dans laquelle
- le capteur vidéo (1) est logé dans l'embout distal (58).

13. Vidéoendoscope selon la revendication 12,
**caractérisé en ce que** le circuit de pré-traitement (3) de signal est :
- logé dans la poignée de commande (51),
- relié au capteur vidéo (1) par un câble électrique multiconducteurs (9) logé dans le tube d'inspection (50, 60), et
- relié au circuit auxiliaire (5) par un câble électrique multiconducteurs (10) logé dans le câble ombilical (44).

14. Vidéoendoscope selon la revendication 13,
**caractérisé en ce que** le circuit auxiliaire (5) est logé dans le dispositif de connexion (40).

15. Vidéoendoscope selon la revendication 13,
**caractérisé en ce que** le circuit auxiliaire (5) est logé dans un coffret externe (85) muni d'une embase de connexion multibroches (84) auquel le dispositif de connexion (40) peut se connecter.

16. Vidéoendoscope selon l'une des revendications 12 à 15,
**caractérisé en ce que** le dispositif- de connexion (40, 76) solidaire de l'extrémité proximale du câble ombilical est équipé de raccords interchangeables (43) pour pouvoir se connecter à différents types de générateurs de lumière.

17. Vidéoendoscope selon l'une quelconque des revendications 1 à 11,
**caractérisé en ce que** le vidéoendoscope est une caméra d'endoscopie comprenant une tête de caméra (70, 90) connectable sur une bonnette (73) d'endoscope ou de fibroscope, la tête de caméra comprenant le capteur vidéo (1), un câble ombilical multiconducteurs (75, 91) dont l'extrémité distale est solidaire à la tête de caméra, et un dispositif de connexion (80, 76, 95) solidaire de l'extrémité proximale du câble ombilical.

18. Vidéoendoscope selon la revendication 17,
**caractérisé en ce que** :
- le circuit de pré-traitement (3) de signal est logé dans la tête de caméra (70), et **en ce que**
- le câble ombilical (75) loge un câble électrique multiconducteurs (10) reliant le circuit de pré-traitement au circuit auxiliaire.

19. Vidéoendoscope selon la revendication 18,
**caractérisé en ce que** le circuit auxiliaire (5) est logé dans un coffret externe (85) muni de moyens (84) de raccordement du dispositif de connexion (76).

20. Vidéoendoscope selon la revendication 18,
**caractérisé en ce que** le circuit auxiliaire (5) est logé dans le dispositif de connexion (80).

21. Vidéoendoscope selon la revendications 17,
**caractérisé en ce que** :
- le circuit de pré-traitement (4) de signal est logé dans le dispositif de connexion (95) relié à la tête de caméra (90) par le câble ombilical (91),
- le circuit auxiliaire (6) est logé dans un coffret externe (100) muni d'une embase de connexion (12), et
- le circuit de pré-traitement (4) de signal est relié au capteur vidéo (1) par un câble électrique multiconducteurs (9) logé dans le câble ombilical, et relié au circuit auxiliaire par un connecteur multibroches (11) solidaire du dispositif de connexion et prévu pour être raccordé à l'embase de connexion (12).

## Claims

1. A videoendoscope comprising at least
- one probe (50, 60, 70) comprising a color video sensor (1),
- one control handle (51, 61) connected to one end of the probe, and
- a video processor comprising :
o a signal preprocessing circuit localized on a preprocessing card (3, 4) and remotely deportable from the video sensor through a first electrical link (15), the preprocessing circuit comprising
■ first signal processing means (13, 14) for generating from the image signals output by the video sensor raw video signals (16, 17) comprising a luminance signal and a chrominance signal, and
■ synchronization means (18, 19) for synchronizing the video sensor and the first signal processing means; and
o an auxiliary circuit localized on an auxiliairy card (5, 6) and comprising second signal processing means connected to the first signal processing means and comprising means (48, 28, 29, 31, 32) for generating from said raw luminance and chrominance video signals at least one useful video signal (30, 31) according to an international standard,
**characterized in that** the videoendoscope further comprises a connection box (40, 80) or an external box (85) connected to the control handle,
**in that**
- the preprocessing card (3, 4) is housed in the control handle (51, 61),
- the raw luminance and chrominance video signals are phase shifted and noisy due to synchronization residues,
- the first electrical link (15) is a high impedance link and is free from any intermediate connection device,
- the auxiliary card (5, 6) is remotely deported from the preprocessing card (3, 4) and is housed in the connection box (40, 80) or in the external box (85), **in that** the auxiliary circuit (5, 6) comprises control means (24) of the digital microcontroller type for configuring said first signal processing means (13, 14), and **in that**
- an umbilical cable (44, 75) is used as housing for a low impedance multiconductor cable (10) providing a good immunity to parasitic currents, electrically connecting the preprocessing card (3, 4) to the auxiliary card (5, 6).

2. The video endoscope according to claim 1,
**characterized in that** the raw chrominance and luminance video signals are of analog nature, the second signal processing means comprising a correction circuit (48) for bringing into phase and filtering the raw luminance and chrominance video signals, and at least one encoding circuit (28, 31) for generating from the brought into phase and filtered raw video signals (49) a useful analog or digital video signal (30, 33) according to an international standard.

3. The video endoscope according to claim 1,
**characterized in that** the raw chrominance and luminance video signals are of digital nature, the second signal processing means comprising at least one correction and encoding circuit (29, 32) for generating from the raw luminance and chrominance video signals a useful analog or digital video signal (30, 33) according to an international standard.

4. The video endoscope according to one of claims 1 to 3, **characterized in that** the video sensor (1) is a color CCD sensor of the line transfer type.

5. The video endoscope according to one of claims 1 to 4, **characterized in that** the signal preprocessing circuit (3, 4) is fixed to the video sensor (1).

6. The video endoscope according to one of claims 1 to 3, **characterized in that** the signal preprocessing circuit (3, 4) is connected through a multiconductor electrical cable (15) to the video sensor (1) that is remotely deported from the preprocessing circuit and comprises phase shifting means (19) for delaying the synchronization signals (20) transmitted to the video sensor (1), to compensate for transmission times induced by the electrical cable (15).

7. The video endoscope according to one of claims 1 to 6, **characterized in that** the signal preprocessing circuit (3, 4) is directly connected to the auxiliary circuit (5, 6) through a multiconductor electrical cable (10), without any intermediate connection device.

8. The video endoscope according to one of claims 1 to 5, **characterized in that** the signal preprocessing circuit (3, 4) is connected to the auxiliary circuit (5, 6) through an electrical link (10) associated with an intermediate connection device (76, 84, 11, 12).

9. The video endoscope according to claim 8,
**characterized in that** the control means (24) are connected to operator command inputting means (7, 8) comprising a command key (34) for triggering configuring the first signal processing means (13, 14) as a function of an illumination color temperature picked up by the video sensor (1).

10. The video endoscope according to one of claims 8 or 9, **characterized in that** the auxiliary circuit (5, 6) comprises a logical interface (25) for connecting the control means (24) to a computer comprising a display screen on which the useful video signal (30, 33) can be displayed, and a keyboard for inputting configuring commands for the first signal processing means (13, 14).

11. The video endoscope according to one of claims 1 to 10, **characterized in that** the auxiliary circuit (5, 6) comprises an electrical power supply circuit (21) for generating from a DC voltage voltages necessary for supplying power to the video sensor (1), the preprocessing circuit (3, 4) and the auxiliary circuit (5, 6).

12. The videoendoscope according to any one of claims 1 to 11, **characterized in that** the videoendoscope is a videoendoscopic probe comprising:
an inspection tube (50, 60) having a distal end which is fixed to a distal end piece (58),
the control handle (51, 61) fixed to the proximal end of the inspection tube,
an umbilical cable (44) the distal end of which is fixed to the control handle,
a connection device (40, 76) fixed to the proximal end of the umbilical cable, and designed to be connected to a light generator, and
a bundle of illumination fibers (45) housed in the umbilical cable, in the control handle and in the inspection tube, the distal end of the bundle being housed in the distal end to illuminate a target when the proximal end of the bundle is connected to a light generator,
in which probe the video sensor (1) is housed in the distal end piece (58).

13. The videoendoscope according to claim 12,
**characterized in that** the signal preprocessing circuit (3) is:
housed in the control handle (51),
connected to the video sensor (1) through a multiconductor electrical cable (9) housed in the inspection tube (50, 60), and
connected to the auxiliary circuit (5) through a multiconductor electrical cable (10) housed in the umbilical cable (44).

14. The videoendoscope according to claim 13,
**characterized in that** the auxiliary circuit (5) is housed in the connection device (40).

15. The videoendoscope according to claim 13,
**characterized in that** the auxiliary circuit (5) is housed in an external box (85) provided with a multi-pin connection socket (84) to which the connection device (40) can be connected.

16. The videoendoscope according to one of claims 12 to 15, **characterized in that** the connection device (40, 76) fixed to the proximal end of the umbilical cable is provided with interchangeable fittings (43) so that it can be connected to different types of light generators.

17. The videoendoscope according to any one of claims 1 to 11, **characterized in that** the videoendoscope is an endoscopy camera comprising a camera head (70, 90) connectable on an eye piece (73) of an endoscope or a fiberscope, the camera head comprising the video sensor (1), a multiconductor umbilical cable (75, 91) of which the distal end is fixed to the camera head, and a connection device (80, 76, 95) fixed to the proximal end of the umbilical cable.

18. The videoendoscope according to claim 17
**characterized in that** :
- the signal preprocessing circuit (3) is housed in the camera head (70), and **in that**
- the umbilical cable (75) houses a multiconductor electrical cable (10) connecting the preprocessing circuit to the auxiliary circuit.

19. The videoendoscope according to claim 18,
**characterized in that** the auxiliary circuit (5) is housed in an external box (85) provided with connection means (84) for connecting the connection device (76).

20. The videoendoscope according to claim 18,
**characterized in that** the auxiliary circuit (5) is housed in the connection device (80).

21. The videoendoscope according to claim 17,
**characterized in that** :
- the signal preprocessing circuit (4) is housed in the connection device (95) connected to the camera head (90) through the umbilical cable (91),
- the auxiliary circuit (6) is housed in an external box (100) provided with a connection socket (12), and
- the signal preprocessing circuit (4) is connected to the video sensor (1) by a multiconductor electrical cable (9) housed in the umbilical cable, and connected to the auxiliary circuit by a multi-pin connector (11) fixed to the connection device and designed to be connected to the connection socket (12).

## Patentansprüche

1. Videoendoskop zumindest umfassend
- eine Sonde (50, 60, 70) umfassend einen Farbvideosensor (1),
- einen mit einer Extremität der Sonde verbundenen Bediengriff (51, 61), und
- einen Videoprozessor umfassend:
+ eine Signalvorverarbeitungsschaltung, welche sich auf einer Vorverarbeitungskarte (3, 4) befindet, und über eine erste elektrische Verbindung (15) beabstandet von dem Videosensor schwenkbar ist, die Vorverarbeitungsschaltung umfassend
* erste Signalverarbeitungsmittel (13, 14) zur Erzeugung von Rohvideosignalen (16, 17) umfassend ein Luminanzsignal und ein Chrominanzsignal ausgehend von den von dem Videosensor gelieferten Bildsignalen, und
* Synchronisationsmittel (18, 19) zur Synchronisation des Videosensors und der ersten Signalverarbeitungsmittel; und
+ eine Hilfsschaltung, welche sich auf einer Hilfskarte (5, 6) befindet, und umfassend zweite Signalverarbeitungsmittel, welche mit den ersten Signalverarbeitungsmitteln verbunden sind, und umfassend Mittel (48, 28, 29, 31, 32) zur Erzeugung zumindest eines nutzbaren einem internationalen Standard entsprechenden Videosignals (30, 33) ausgehend von Luminanz- und Chrominanzrohvideosignalen,
**dadurch gekennzeichnet, dass** das Videoendoskop zudem umfasst ein Verbindungsgehäuse (40, 80) oder ein externes Gehäuse (85) verbunden mit dem Bediengriff,
wobei
- die Vorverarbeitungskarte (3, 4) in dem Bediengriff (51, 61) angeordnet ist,
- die Chrominanz- und Luminanzrohvideosignale phasenverschoben und durch Reste der Synchronisation verrauscht sind,
- die erste elektrische Verbindung (15) eine hohe Impedanz aufweist und bar jeder Zwischenverbindungseinrichtung ist,
- die Hilfskarte (5, 6) um eine Strecke von der Vorverarbeitungskarte (3, 4) verschoben ist, und in dem Verbindungsgehäuse (40, 80) oder in dem externen Gehäuse (85) angeordnet ist,
- wobei die Hilfsschaltung (5, 6) Steuerungsmittel (24) vom Typ numerischer Mikrocontroller umfasst, um die ersten Signalverarbeitungsmittel (13, 14) zu parametrisieren, und wobei
- ein Anbindungskabel (44, 75) als Unterbringung eines mehrsträngigen Kabels (10) geringer Impedanz dient, was eine gute Abwehr gegen Störungen bietet, wobei die Vorverarbeitungskarte (3, 4) mit der Hilfskarte (5, 6) elektrisch verbunden ist.

2. Videoendoskop nach Anspruch 1, **dadurch gekennzeichnet, dass** die Chrominanz- und Luminanzrohvideosignale analoger Natur sind, die zweiten Signalverarbeitungsmittel eine Korrekturschaltung (48) umfassen, um die Luminanz- und Chrominanzrohvideosignale in Phase zu bringen und zu filtern, und zumindest eine Kodierschaltung (28, 31) zur Erzeugung eines analogen oder numerischen (30, 33) nutzbaren einem internationalen Standard entsprechenden Videosignals ausgehend von in Phase gebrachten und gefilterten Rohvideosignalen (49) umfassen.

3. Videoendoskop nach Anspruch 1, **dadurch gekennzeichnet, dass** die Chrominanz- und Luminanzrohvideosignale numerischer Natur sind, die zweiten Signalverarbeitungsmittel umfassend zumindest eine Korrektur- und Kodierschaltung (29, 32) zur Erzeugung eines analogen oder numerischen (30, 33) nutzbaren einem internationalen Standard entsprechenden Videosignals ausgehend von Luminanz- und Chrominanzrohvideosignalen.

4. Videoendoskop nach einem der Ansprüche 1 bis 3, **dadurch gekennzeichnet, dass** der Videosensor (1) ein auf zeilenweisem Transfer basierender CCD Farbsensor ist.

5. Videoendoskop nach einem der Ansprüche 1 bis 4, **dadurch gekennzeichnet, dass** die Signalvorverarbeitungsschaltung (3, 4) mit dem Videosensor (1) einstückig ausgebildet ist.

6. Videoendoskop nach einem der Ansprüche 1 bis 3, **dadurch gekennzeichnet, dass** die Signalvorverarbeitungsschaltung (3, 4) über ein elektrisches mehrsträngiges Kabel (15) mit dem Videosensor (1) verschaltet ist, welcher sich um eine Strecke von der Vorverarbeitungsschaltung verschoben befindet, und Phasenverschiebungsmittel (19) zur Verzögerung der zum Videosensor (1) übertragenen Synchronisationssignale (20) umfasst, um Verzögerungen der Übertragung zu kompensieren, die durch das elektrische Kabel eingeführt werden (15).

7. Videoendoskop nach einem der Ansprüche 1 bis 6, **dadurch gekennzeichnet, dass** die Signalvorverarbeitungsschaltung (3, 4) direkt mit der Hilfsschaltung (5, 6) über ein mehrsträngiges elektrisches Kabel (10) verbunden ist, ohne Zwischenverbindungseinrichtung.

8. Videoendoskop nach einem der Ansprüche 1 bis 5, **dadurch gekennzeichnet, dass** die Signalvorverarbeitungsschaltung (3, 4) mit der Hilfsschaltung (5, 6) über eine elektrische Verbindung (10) verbunden ist, assoziiert mit einer Zwischenverbindungseinrichtung (76, 84, 11, 12).

9. Videoendoskop nach Anspruch 8, **dadurch gekennzeichnet, dass** die Steuerungsmittel (24) mit Eingabemitteln (7, 8) für Kommandos einer Bedienperson verbunden sind umfassend eine Funktionstaste (34) zum Auslösen der Parametrisierung der ersten Signalverarbeitungsmittel (13, 14) als Funktion einer mit dem Videosensor (1) festgestellten Beleuchtungsfarbtemperatur.

10. Videoendoskop nach einem der Ansprüche 8 bis 9, **dadurch gekennzeichnet, dass** die Hilfsschaltung (5, 6) eine logische Schnittstelle (25) umfasst, welche es erlaubt, die Steuerungsmittel (24) mit einem einen Bildschirm aufweisenden Rechner zu verbinden, auf dem das nutzbare Videosignal (30, 33) gezeigt werden kann, und eine Tastatur, welche es erlaubt, Kommandos für die Parametrisierung der ersten Signalverarbeitungsmittel (13, 14) einzugeben.

11. Videoendoskop nach einem der Ansprüche 1 bis 10, **dadurch gekennzeichnet, dass** die Hilfsschaltung (5, 6) eine elektrische Versorgungsschaltung (21) umfasst zur Erzeugung notwendiger Spannungen für die Versorgung des Videosensors (1), der Vorverarbeitungsschaltung (3, 4) und der Hilfsschaltung (5, 6) ausgehend von einer kontinuierlichen Spannung.

12. Videoendoskop nach einem der Ansprüche 1 bis 11,
**dadurch gekennzeichnet, dass** das Videoendoskop eine videoendoskopische Sonde ist, umfassend:
- einen Inspektionstubus (50, 60), dessen distales Endstück mit einer distalen Endabdeckung (58) einstückig ausgebildet ist,
- den Bediengriff (51, 61) einstückig ausgebildet mit dem proximalen Endstück des Inspektionstubus,
- ein Anbindungskabel (44), dessen distales Endstück mit dem Bediengriff einstückig ausgebildet ist,
- eine Verbindungseinrichtung (40, 76) einstückig ausgebildet mit dem proximalen Endstück des Anbindungskabels, und dazu ausgelegt, an einen Beleuchtungsgenerator angeschlossen zu sein, und
- ein Bündel Beleuchtungsfasern (45), untergebracht in dem Anbindungskabel, in dem Bediengriff, und in dem Inspektionstubus, wobei das distale Endstück des Bündels in der distalen Endabdeckung zum Beleuchten eines Ziels untergebracht ist, wenn das proximale Endstück des Bündels an einen Beleuchtungsgenerator angeschlossen ist,
- Sonde in der der Videosensor (1) in der distalen Endabdeckung (58) untergebracht ist.

13. Videoendoskop nach Anspruch 12, **dadurch gekennzeichnet, dass** die Signalvorverarbeitungsschaltung (3):
- in dem Bediengriff (51) untergebracht ist,
- verbunden ist mit dem Videosensor (1) über ein mehrsträngiges elektrisches Kabel (9) untergebracht in dem Inspektionstubus (50, 60), und
- verbunden ist mit der Hilfsschaltung (5) über ein mehrsträngiges elektrisches Kabel (10) untergebracht in dem Anbindungskabel (44).

14. Videoendoskop nach Anspruch 13, **dadurch gekennzeichnet, dass** die Hilfsschaltung (5) in der Verbindungseinrichtung (40) untergebracht ist.

15. Videoendoskop nach Anspruch 13, **dadurch gekennzeichnet, dass** die Hilfsschaltung (5) in einem externen Gehäuse (85) untergebracht ist, versehen mit einem Multipinsockel (84), an den die Verbindungseinrichtung (40) angeschlossen werden kann.

16. Videoendoskop nach einem der Ansprüche 12 bis 15, **dadurch gekennzeichnet, dass** die Verbindungseinrichtung (40, 76) im Zusammenhalt mit dem proximalen Endstück des Anbindungskabels ausgestattet ist mit einer austauschbaren Verbindung (43), um an unterschiedliche Typen von Beleuchtungsgeneratoren angeschlossen werden zu können.

17. Videoendoskop nach einem der Ansprüche 1 bis 11, **dadurch gekennzeichnet, dass** das Videoendoskop eine Endoskopiekamera ist umfassend einen Kamerakopf (70, 90) anschließbar an eine Vorsatzlinse (73) eines Endoskops oder Fibroskops, der Kamerakopf umfassend den Videosensor (1), ein mehrsträngiges Anbindungskabel (75, 91) dessen distales Endstück mit dem Kamerakopf einstückig ausgebildet ist, und eine Verbindungseinrichtung (80, 76, 95) in einstückiger Ausbildung mit dem proximalen Endstück des Anbindungskabels.

18. Videoendoskop nach Anspruch 17, **dadurch gekennzeichnet, dass**:
- die Signalvorverarbeitungsschaltung (3) in dem Kamerakopf (70) untergebracht ist, und wobei
- das Anbindungskabel (75) ein mehrsträngiges elektrisches Kabel (10) unterbringt, welches die Vorverarbeitungsschaltung mit der Hilfsschaltung verbindet.

19. Videoendoskop nach Anspruch 18, **dadurch gekennzeichnet, dass** die Hilfsschaltung (5) in einem externen Gehäuse (85) versehen mit Mitteln (84) zum Anschließen der Verbindungseinrichtung (76) untergebracht ist.

20. Videoendoskop nach Anspruch 18, **dadurch gekennzeichnet, dass** die Hilfsschaltung (5) in der Verbindungseinrichtung (80) untergebracht ist.

21. Videoendoskop nach Anspruch 17, **dadurch gekennzeichnet, dass**:
- die Signalvorverarbeitungsschaltung (4) in der Verbindungseinrichtung (95) untergebracht ist, verbunden mit dem Kamerakopf (90) über das Anbindungskabel (91),
- die Hilfsschaltung (6) in einem externen Gehäuse (100) versehen mit einem Anschlusssockel (12) untergebracht ist, und
- die Signalvorverarbeitungsschaltung (4) mit dem Videosensor (1) verbunden ist über ein elektrisches mehrsträngiges Kabel (9) untergebracht in dem Anbindungskabel, und verbunden mit der Hilfsschaltung über einen Multipinstecker (11) im Zusammenhalt mit der Verbindungseinrichtung und dazu ausgelegt, an den Verbindungssockel (12) angeschlossen zu sein.
